# EUROPEAN PATENT APPLICATION

(11) **EP 3 173 095 A1**
(43) Date of publication of application: **31.05.2017**
(21) Application number: 16205767.3
(22) Date of filing: 13.10.2011
(51) Int. Cl.: A61K 38/48, A61K 47/50, A61P 29/00, A61P 25/06

(54) **TARGETED DELIVERY OF TARGETED EXOCYTOSIS MODULATORS TO THE SPHENOPALATINE GANGLION FOR TREATMENT OF HEADACHE DISORDERS**

(30) Priority: 14.10.2010 US 393266 P
(62) Divisional of application: 11776960.4
(71) Applicant: ALLERGAN, INC., Irvine, CA 92612 (US)
(72) Inventor: Blumenfeld, Andrew M., Del Mar, CA California 92014 (US)
(74) Representative: Hoffmann Eitle

(57) **Abstract**

The present specification discloses the use of Targeted Exocytosis Modulators to the sphenopalatine ganglion to treat headache disorders.

## Description

### CROSS REFERENCE

This application claims the benefit of U.S. Provisional Patent Application Serial Number 61/393,266, filed on October 14, 2010, the entire disclosure of which is incorporated herein by this specific reference.

The present invention generally relates to the use of Targeted Exocytosis Modulators to the sphenopalatine ganglion to treat headache disorders.

### SUMMARY

Migraine is a primary headache disorder that may be characterized by unilateral throbbing pain which worsens with head movement. This can be associated with other symptoms including nausea, light and noise sensitivity, lacrimation, nasal congestion, and rhinorrhea. An array of factors can trigger migraine headache, such as internal changes (hormonal changes, stress, sleep deprivation) or external changes (weather changes, alcohol, flickering light).

In some cases, a migraine attack begins with a premonitory visual aura. These patients experience a visual disturbance in the form of a zigzag spectrum around a blind spot, which grows in size over a 20-30 min period. This visual effect is known as the "fortification spectrum." The development of the fortification spectrum over time has been shown to correspond to a wave of depression in the activity of cortical neurons, which typically begins in the occipital lobe, and spreads anteriorly. The establishment of this correspondence has permitted the elaboration of a theory about the pathophysiological changes that may cause migraine and other headaches.

As neurons depress, they release nitric oxide (NO), which triggers the dilation of meningeal blood vessels. This vasodilation can result in a dull headache, which corresponds to the earliest phase of migraine.

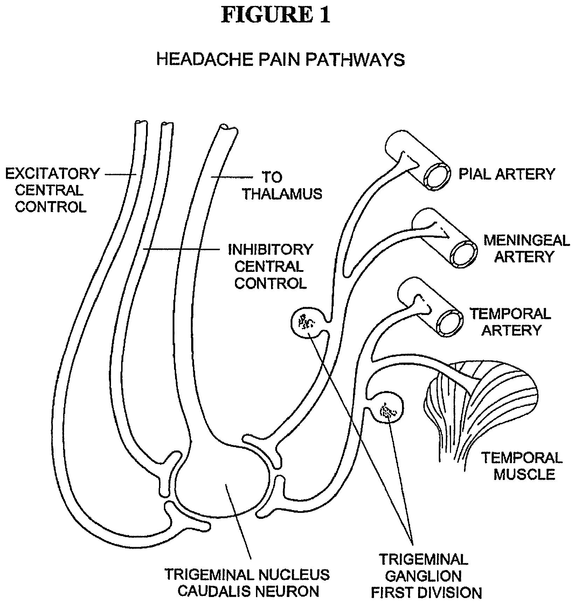

The dilation of the meningeal blood vessels increases the activity of the nerve endings of the primary afferent neurons of the trigeminal nerve that are wrapped around them. As a result, the trigeminal cells release calcitonin gene related protein (CGRP), a vasodilator neuropeptide which further increases the dilation of the meningeal blood vessels, and further feeds into the trigeminal nerve activation. The local intracranial increased activation of the trigeminal nerve spreads through the trigeminal ganglion into the Trigeminal Nucleus Caudalis (TNC) in the brainstem in a process known as peripheral sensitization. The activation of the TNC leads in turn to a central activation process, through its thalamic and cortical projections, which are illustrated in FIG. 1.

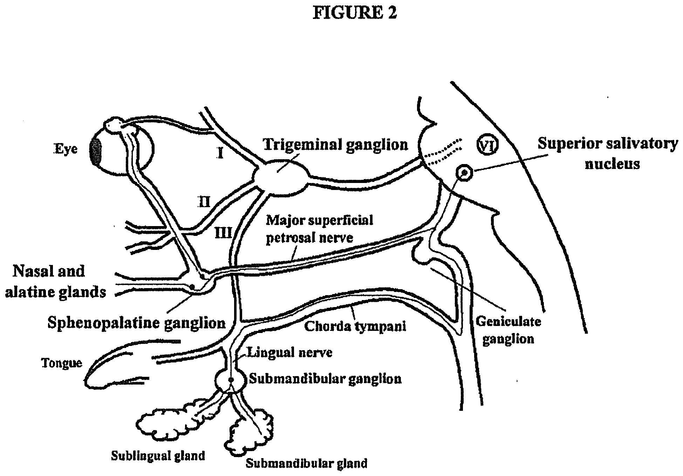

Although the pain associated with migraine involves input from meningeal arteries, activation of the TNC may result in referred pain anywhere along the trigeminal network, including the temporal arteries and temporal muscles. The trigeminocervical network involved in the pathophysiology of migraine contains the three main branches of the trigeminal nerve: the ophthalmic branch (V1), the maxillary branch (V2), and the mandibular branch (V3), as illustrated in FIG. 2; as well as the sensory nerves for the posterior head and neck (C2, C3, C4, C5) that feed into the TNC. A detailed anatomical map of the relevant pathways can be found in pages 316, 317, 600, 601 and 736 of Agur, A. M. R. and Dalley II, A. F. (2005) Atlas of Anatomy 11th Ed., Lippincottt Williams & Wilkins, Philadelphia, which is hereby incorporated by reference.

The activation of the TNC in the brainstem can further spread to the occipital nerve by virtue of its anatomical connection to the TNC, leading to pain sensation in the occipital area.

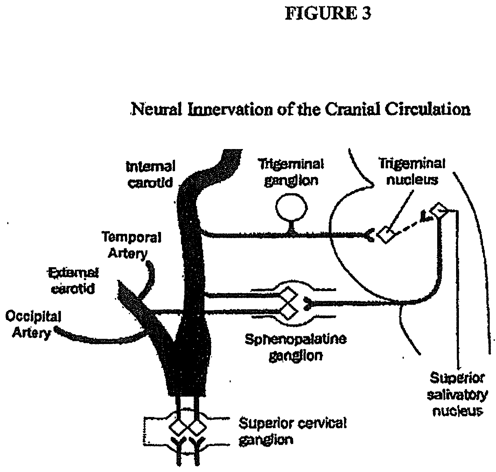

The activation of the TNC can also spread to the parasympathetic system, by activation of a nearby nucleus in the brainstem, the Superior Salivatory Nucleus (SSN), which is connected to the nucleus caudalis through a network of interneurons, as shown in FIG. 3.

Neurons from the SSN synapse with the Sphenopalatine ganglion, which provides vasomotor innervation to blood vessels and secretomotor innervation to the lacrimal glands, nasal and sinus mucosa. When the parasympathetic system is activated, the upper respiratory tract symptoms associated with migraine occur including, potentially, nasal symptoms (rhinorrhea, and post nasal drip), ocular symptoms (conjunctival injection, and tearing) and sinus congestion (pain or pressure around the sinuses). Other parasympathetic projections further aggravate the cascade of events, like the Sphenopalatine ganglion afferents that innervate the meningeal blood vessels. Activation of the parasympathetic system during a migraine attack is also accompanied by a significant increase in the levels of Vasoactive Intestinal Polypeptide (VIP), a parasympathetic neurotransmitter which causes vasodilation and can be measured in high concentrations during a migraine in the jugular venous drainage.

The increased activity of the trigeminal, occipital and parasympathetic systems just described is common to the so-called Trigeminal Autonomic Cephalgias (TAC), which include Cluster headache, Paroxysmal Hemicrania, SUNCT Syndrome, and Hemicrania Continua. Cluster headaches are a primary headache disorder involving attacks of less than 3 hours of duration with severe unilateral peri-orbital and temporal pain. These headaches can be associated with lacrimation, nasal congestion, rhinorrhea, conjunctival injection and a Horner's syndrome. The attacks occur in distinct clusters. Cluster headaches typically involve a series of disabling attacks on a daily basis lasting for months at a time. This pattern recurs annually or biannually.

The trigeminal nerve is involved in the pain sensations for all of these headache types, as well as headaches triggered by other pathologies. For example, Temporal Arteritis involves inflammation of the temporal artery with painful palpable nodules along the artery. In addition to headache in the temporal area, Temporal Arteritis causes vision loss and jaw pain.

Headaches can also be associated with ischemic stroke. In a stroke, a lack of blood supply to brain tissue causes a sudden localized neurological deficit. In a large number of affected patients, occlusion of the arteries is due to the presence of atherosclerotic plaques in the arteries supplying the brain, for example, the carotid artery and the vertebral basilar artery. The atherosclerotic plaques are often associated with inflammation which further contributes to occlusion of the blood vessel.

Nociceptive fibers stimulated by inflammatory mediators in infectious or allergic rhinitis can also activate the trigeminal brainstem nucleus and precipitate migraine.

TAC and migraine are difficult to treat. Numerous medications have been used to prevent cluster and migraine headaches from occurring, which include, amongst others: propranolol, timolol, divalproex sodium, topiramate, verapamil, indomethacin and amitriptyline. These medicines have numerous side effects and patients are poorly compliant with them. In the case of TAC, indomethacin, in particular, is difficult for patients to tolerate due to gastro-intestinal upset.

All of the headache disorders described above produce disability and better treatment modalities are needed.

Recently, Botulinum toxin has been shown to be effective to treat migraine headaches when injected in the face, cranium and neck (Binder, U.S. Pat. No. 5,714,468 and Blumenfeld, U.S. Patent 7,981,433, both incorporated entirely by reference). Botulinum toxin is a potent polypeptide neurotoxin produced by the gram positive bacterium Clostridium botulinum which causes a paralytic illness in humans termed botulism. Botulinum toxin has a light and a heavy chain. The heavy chain attaches to a cell surface receptor and the complex is then endocytosed. After endocytosis, the light chain translocates from the endosome into the cytoplasm, where it cleaves a segment of the SNARE protein complex responsible for vesicle fusion in the presynaptic nerve terminal. As a result, the release of neurotransmitters from these vesicles is effectively blocked for 3-6 months..

Aspects of the present specification disclose methods of treating a headache disorders in a human, the methods comprising the step of administering to the human in need thereof a therapeutically effective amount of a composition including a TEM, wherein administration of the composition reduces a symptom of the migraine disorder, thereby treating the human. In some aspects, a TEM may comprise a targeting domain, a Clostridial toxin translocation domain and a Clostridial toxin enzymatic domain. In some aspects, a TEM may comprise a targeting domain, a Clostridial toxin translocation domain, a Clostridial toxin enzymatic domain, and an exogenous protease cleavage site. A targeting domain includes, without limitation, a sensory neuron targeting domain, a sympathetic neuron targeting domain, or a parasympathetic neuron targeting domain. Headache disorders include, without limitation, a migraine without aura, a migraine with aura, a menstrual migraine, a migraine equivalent, a complicated migraine, an abdominal migraine, or a mixed tension migraine.

Other aspects of the present specification disclose uses of a TEM disclosed herein in the manufacturing a medicament for treating a migraine disorder disclosed herein in a human in need thereof.

### BRIEF DESCRIPTION OF THE DRAWINGS

FIG. 1 depicts headache pain pathways, illustrating the input and output pathways of the trigeminal nucleus caudalis in the brainstem.
FIG. 2 depicts the trigeminal and parasympathetic network involved in headache pain, from and into the brainstem.
FIG. 3 depicts the neural innervation of the cranial circulation, illustrating the trigeminal nociceptive input from the internal carotid to the trigeminal nucleus caudalis of the brainstem, and the connection to the parasympathetic system through the superior salivatory nucleus. The parasympathetic system feeds back into the internal and external carotids, which connect with the temporal and occipital arteries.
FIG. 4 depicts an illustrated lateral view of a human head showing the connection between the external carotid and the superficial temporal and occipital arteries. FIG. 4 has been adapted from Agur, A. M. R. and Dalley II, A. F. (2005) Atlas of Anatomy 11.sup.th Ed., Lippincottt Williams & Wilkins, Philadelphia. Refer to pages 316, 317, 600, 601 and 736 of the original reference for a detailed view of the anatomy.

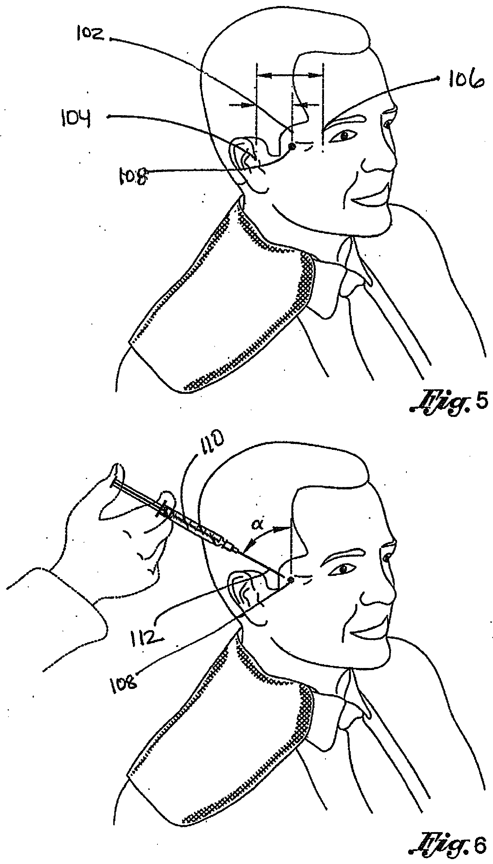

FIG. 5 depicts mapping of the anterior temporal fossa.
FIG. 6 depicts injection of a TEM by insertion of a needle at angle α.

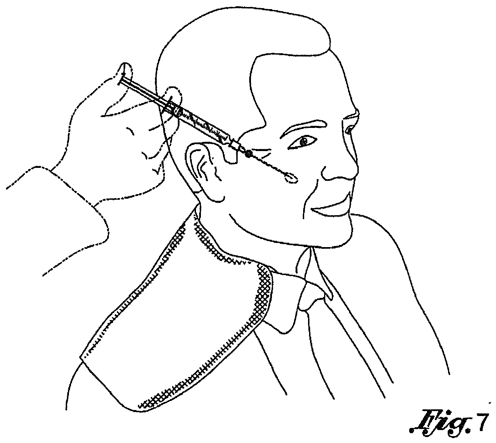

FIG. 7 depicts one embodiment of the disclosed method wherein discontinuous discreet injections of TEM are administered to or around the sphenopalatine ganglion.

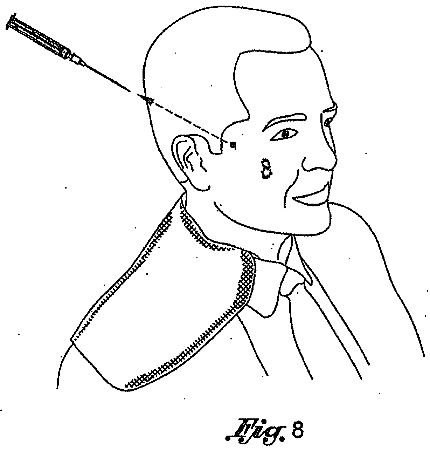

FIG. 8 depicts the withdrawal of the needle along an injection track.

### DESCRIPTION

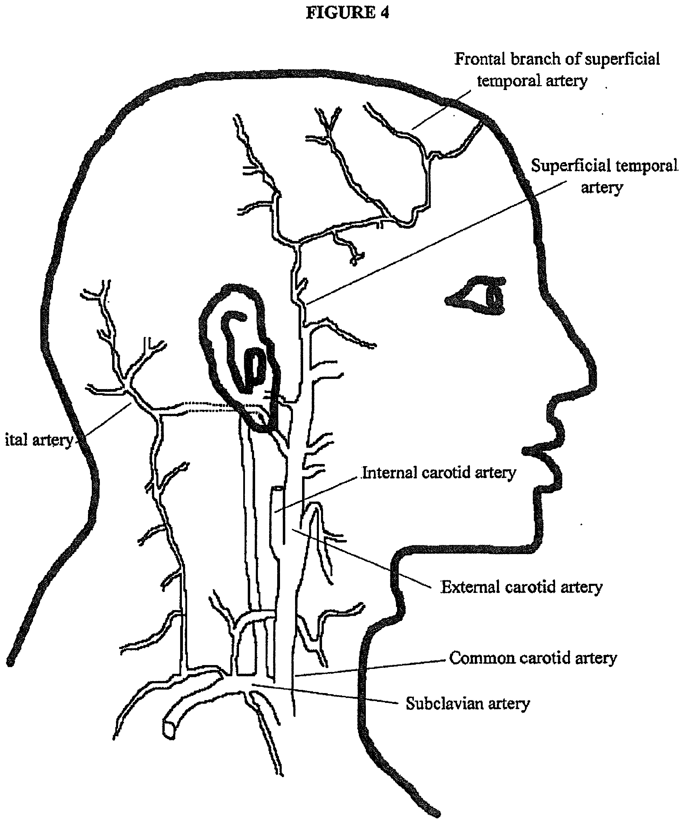

[The anatomical and physiological theory of migraine, trigeminal autonomic cephalgias (TAC), and other headaches associated with vascular conditions, as described in the Background section, suggests that blockade of the release of nociceptive and inflammatory agents triggered by the hyperactivation of the trigeminal, occipital and parasympathetic systems involved in the development of these headaches should provide an effective therapeutic and/or prophylactic treatment. As depicted in FIGS. 1 and 2, some of the anatomical pathways involved in the development of these headaches are intracranial; therefore, specific blockade of the intracranial pathways involved by non-invasive means is not feasible. However, some of the pathways affected are located extracranially under the surface of the skin or intranasally, and are therefore accessible to treatment. Some examples of these pathways include the temporal arteries and muscles (FIGS. 1 and 4), the nasal glands and mucosa (FIG. 2) and the occipital nerve and artery (FIG. 4). The current disclosure relates to providing a method for blocking the release of nociceptive and inflammatory agents triggered by the hyperactivation of the trigeminal, occipital and parasympathetic fibers that is increased during migraine, TAC or other headaches associated with vascular conditions.

Clostridia toxins produced by *Clostridium botulinum, Clostridium tetani, Clostridium baratii* and *Clostridium butyricum* are the most widely used in therapeutic and cosmetic treatments of humans and other mammals. Strains of *C*. *botulinum* produce seven antigenically-distinct types of Botulinum toxins (BoNTs), which have been identified by investigating botulism outbreaks in man (BoNT/A, BoNT/B, BoNT/E and BoNT/F), animals (BoNT/C1 and BoNT/D), or isolated from soil (BoNT/G). BoNTs possess approximately 35% amino acid identity with each other and share the same functional domain organization and overall structural architecture. It is recognized by those of skill in the art that within each type of Clostridial toxin there can be subtypes that differ somewhat in their amino acid sequence, and also in the nucleic acids encoding these proteins. For example, there are presently five BoNT/A subtypes, BoNT/A1, BoNT/A2, BoNT/A3 BoNT/A4 and BoNT/A5, with specific subtypes showing approximately 89% amino acid identity when compared to another BoNT/A subtype. While all seven BoNT serotypes have similar structure and pharmacological properties, each also displays heterogeneous bacteriological characteristics. In contrast, tetanus toxin (TeNT) is produced by a uniform group of *C*. *tetani.* Two other Clostridia species, *C*. *baratii* and *C*. *butyricum,* produce toxins, BaNT and BuNT, which are functionally similar to BoNT/F and BoNT/E, respectively.

Each mature di-chain molecule of a Clostridial toxin comprises three functionally distinct domains: 1) an enzymatic domain located in the light chain (LC) that includes a metalloprotease region containing a zinc-dependent endopeptidase activity which specifically targets core components of the neurotransmitter release apparatus; 2) a translocation domain contained within the amino-terminal half of the heavy chain (H_{N}) that facilitates release of the LC from intracellular vesicles into the cytoplasm of the target cell; and 3) a binding domain found within the carboxyl-terminal half of the heavy chain (H_{C}) that determines the binding activity and binding specificity of the toxin to the receptor complex located at the surface of the target cell. The H_{C} domain comprises two distinct structural features of roughly equal size that indicate function and are designated the H_{CN} and H_{CC} subdomains.

Clostridial toxins act on the nervous system by blocking the release of acetylcholine (ACh) at the pre-synaptic neuromuscular junction. The binding, translocation and enzymatic activity of these three functional domains are all necessary for toxicity. While all details of this process are not yet precisely known, the overall cellular intoxication mechanism whereby Clostridial toxins enter a neuron and inhibit neurotransmitter release is similar, regardless of serotype or subtype. Although applicants have no wish to be limited by the following description, the intoxication mechanism can be described as comprising at least four steps: 1) receptor binding, 2) complex internalization, 3) light chain translocation, and 4) enzymatic target modification. The process is initiated when the binding domain of a Clostridial toxin binds to a toxin-specific receptor system located on the plasma membrane surface of a target cell. The binding specificity of a receptor complex is thought to be achieved, in part, by specific combinations of gangliosides and protein receptors that appear to distinctly comprise each Clostridial toxin receptor complex. Once bound, the toxin/receptor complexes are internalized by endocytosis and the internalized vesicles are sorted to specific intracellular routes. The translocation step appears to be triggered by the acidification of the vesicle compartment. This process seems to initiate pH-dependent structural rearrangements that increase hydrophobicity, create a pore in the vesicle membrane, and promote formation of the di-chain form of the toxin. Once di-chain formation occurs, light chain endopeptidase of the toxin is released from the intracellular vesicle via the pore into the cytosol where it appears to specifically target one of three known core components of the neurotransmitter release apparatus. These core proteins, vesicle-associated membrane protein (VAMP)/synaptobrevin, synaptosomal-associated protein of 25 kDa (SNAP-25) and Syntaxin, are necessary for synaptic vesicle docking and fusion at the nerve terminal and constitute members of the soluble *N*-ethylmaleimide-sensitive factor-attachment protein-receptor (SNARE) family. BoNT/A and BoNT/E cleave SNAP-25 in the carboxyl-terminal region, releasing a nine or twenty-six amino acid segment, respectively, and BoNT/C1 also cleaves SNAP-25 near the carboxyl-terminus. The botulinum serotypes BoNT/B, BoNT/D, BoNT/F and BoNT/G, and tetanus toxin, act on the conserved central portion of VAMP, and release the amino-terminal portion of VAMP into the cytosol. BoNT/C1 cleaves syntaxin at a single site near the cytosolic membrane surface.

A Clostridial toxin treatment inhibits neurotransmitter release by disrupting the exocytotic process used to secret the neurotransmitter into the synaptic cleft. There is a great desire by the pharmaceutical industry to expand the use of Clostridial toxin therapies beyond its current myo-relaxant applications to treat sensory nerve-based ailment, such as, e.g., various kinds of chronic pain, neurogenic inflammation and urogentital disorders, as well as other disorders, such as, e.g., pancreatitis. One approach to expand the use of Clostridial toxin-based therapies involves modifying a Clostridial toxin so that the modified toxin has an altered cell targeting capability. This re-targeted capability is achieved by replacing a naturally-occurring targeting domain of a Clostridial toxin with a targeting domain having a binding activity for a non-Clostridial toxin receptor. Called Targeted Exocytosis Modulator (TEMs), these retargeted molecules bind to a non-Clostridial toxin receptor, internalize into the cytoplasm, translocate the enzymatic domain into the cytoplasm, and exert a proteolytic effect on a component of the SNARE complex of the target cell.

An important difference between TEMs, such as, *e.g*., TEMs disclosed herein, and native Clostridial toxins is that since TEMs do not target motor neurons, the lethality associated with over-dosing a human with a TEM is greatly minimized, if not avoided altogether. For example, a TEM comprising an opioid targeting domain can be administered at 10,000 times the therapeutically effective dose before evidence of lethality is observed, and this lethality is due to the passive diffusion of the molecule and not via the intoxication process. Thus, for all practical purposes TEMs are non-lethal molecules.

Aspects of the present disclosure comprise, in part, a Targeted Vesicular Exocytosis Modulator Protein. As used herein, the term Targeted Vesicular Exocytosis Modulator Protein" is synonymous with "TEM" or "retargeted endopeptidase." Generally, a TEM comprises an enzymatic domain from a Clostridial toxin light chain, a translocation domain from a Clostridial toxin heavy chain, and a targeting domain. The targeting domain of a TEM provides an altered cell targeting capability that targets the molecule to a receptor other than the native Clostridial toxin receptor utilized by a naturally-occurring Clostridial toxin. This re-targeted capability is achieved by replacing the naturally-occurring binding domain of a Clostridial toxin with a targeting domain having a binding activity for a non-Clostridial toxin receptor. Although binding to a non-Clostridial toxin receptor, a TEM undergoes all the other steps of the intoxication process including internalization of the TEM/receptor complex into the cytoplasm, formation of the pore in the vesicle membrane and di-chain molecule, translocation of the enzymatic domain into the cytoplasm, and exerting a proteolytic effect on a component of the SNARE complex of the target cell.

As used herein, the term "Clostridial toxin enzymatic domain" refers to a Clostridial toxin polypeptide located in the light chain of a Clostridial toxin that executes the enzymatic target modification step of the intoxication process. A Clostridial toxin enzymatic domain includes a metalloprotease region containing a zinc-dependent endopeptidase activity which specifically targets core components of the neurotransmitter release apparatus. Thus, a Clostridial toxin enzymatic domain specifically targets and proteolytically cleavages of a Clostridial toxin substrate, such as, e.g., SNARE proteins like a SNAP-25 substrate, a VAMP substrate and a Syntaxin substrate.

A Clostridial toxin enzymatic domain includes, without limitation, naturally occurring Clostridial toxin enzymatic domain variants, such as, e.g., Clostridial toxin enzymatic domain isoforms and Clostridial toxin enzymatic domain subtypes; non-naturally occurring Clostridial toxin enzymatic domain variants, such as, e.g., conservative Clostridial toxin enzymatic domain variants, non-conservative Clostridial toxin enzymatic domain variants, Clostridial toxin enzymatic domain chimeras, active Clostridial toxin enzymatic domain fragments thereof, or any combination thereof. Non-limiting examples of a Clostridial toxin enzymatic domain include, *e.g.,* a BoNT/A enzymatic domain, a BoNT/B enzymatic domain, a BoNT/C1 enzymatic domain, a BoNT/D enzymatic domain, a BoNT/E enzymatic domain, a BoNT/F enzymatic domain, a BoNT/G enzymatic domain, a TeNT enzymatic domain, a BaNT enzymatic domain, and a BuNT enzymatic domain.

As used herein, the term "Clostridial toxin translocation domain" refers to a Clostridial toxin polypeptide located within the amino-terminal half of the heavy chain of a Clostridial toxin that executes the translocation step of the intoxication process. The translocation step appears to involve an allosteric conformational change of the translocation domain caused by a decrease in pH within the intracellular vesicle. This conformational change results in the formation of a pore in the vesicular membrane that permits the movement of the light chain from within the vesicle into the cytoplasm. Thus, a Clostridial toxin translocation domain facilitates the movement of a Clostridial toxin light chain across a membrane of an intracellular vesicle into the cytoplasm of a cell.

A Clostridial toxin translocation domain includes, without limitation, naturally occurring Clostridial toxin translocation domain variants, such as, e.g., Clostridial toxin translocation domain isoforms and Clostridial toxin translocation domain subtypes; non-naturally occurring Clostridial toxin translocation domain variants, such as, e.g., conservative Clostridial toxin translocation domain variants, non-conservative Clostridial toxin translocation domain variants, Clostridial toxin translocation domain chimerics, active Clostridial toxin translocation domain fragments thereof, or any combination thereof. Non-limiting examples of a Clostridial toxin translocation domain include, *e.g.,* a BoNT/A translocation domain, a BoNT/B translocation domain, a BoNT/C1 translocation domain, a BoNT/D translocation domain, a BoNT/E translocation domain, a BoNT/F translocation domain, a BoNT/G translocation domain, a TeNT translocation domain, a BaNT translocation domain, and a BuNT translocation domain.

As used herein, the term "targeting domain" is synonymous with "binding domain" or "targeting moiety" and refers to a polypeptide that executes the receptor binding and/or complex internalization steps of the intoxication process, with the proviso that the binding domain is not a Clostridial toxin binding domain found within the carboxyl-terminal half of the heavy chain of a Clostridial toxin. A targeting domain includes a receptor binding region that confers the binding activity and/or specificity of the targeting domain for its cognate receptor. As used herein, the term "cognate receptor" refers to a receptor for which the targeting domain preferentially interacts with under physiological conditions, or under in vitro conditions substantially approximating physiological conditions. As used herein, the term "preferentially interacts" is synonymous with "preferentially binding" and refers to an interaction that is statistically significantly greater in degree relative to a control. With reference to a targeting domain disclosed herein, a targeting domain binds to its cognate receptor to a statistically significantly greater degree relative to a non-cognate receptor. Said another way, there is a discriminatory binding of the targeting domain to its cognate receptor relative to a non-cognate receptor. Thus, a targeting domain directs binding to a TEM-specific receptor located on the plasma membrane surface of a target cell.

In an embodiment, a targeting domain disclosed herein has an association rate constant that confers preferential binding to its cognate receptor. In aspects of this embodiment, a targeting domain disclosed herein binds to its cognate receptor with an association rate constant of, e.g., less than 1 x 10⁵ M⁻¹ s⁻¹, less than 1 x 10⁶ M⁻¹ s⁻¹, less than 1 x 10⁷ M⁻¹ s⁻¹, or less than 1 x 10⁶ M⁻¹ s⁻¹. In other aspects of this embodiment, a targeting domain disclosed herein binds to its cognate receptor with an association rate constant of, e.g., more than 1 x 10⁵ M⁻¹ s⁻¹, more than 1 x 10⁶ M⁻¹ s⁻¹, more than 1 x 10⁷ M⁻¹ s⁻¹, or more than 1 x 10⁸ M⁻¹ s⁻¹. In yet other aspects of this embodiment, a targeting domain disclosed herein binds to its cognate receptor with an association rate constant between 1 x 10⁵ M⁻¹ s⁻¹ to 1 x 10⁸ M⁻¹ s⁻¹, 1 x 10⁶ M⁻¹ s⁻¹ to 1 x 10⁸ M⁻¹ s⁻¹, 1 x 10⁵ M⁻¹ s⁻¹ to 1 x 10⁷ M⁻¹ s⁻¹, or 1 x 10⁶ M⁻¹ s⁻¹ to 1 x 10⁷ M⁻¹ s⁻¹.

In another embodiment, a targeting domain disclosed herein has an association rate constant that is greater for its cognate target receptor relative to a non-cognate receptor. In other aspects of this embodiment, a targeting domain disclosed herein has an association rate constant that is greater for its cognate target receptor relative to a non-cognate receptor by, at least one-fold, at least two-fold, at least three-fold, at least four fold, at least five-fold, at least 10 fold, at least 50 fold, at least 100 fold, at least 1000 fold, at least 10,000 fold, or at least 100,000 fold. In other aspects of this embodiment, a targeting domain disclosed herein has an association rate constant that is greater for its cognate target receptor relative to a non-cognate receptor by, e.g., about one-fold to about three-fold, about one-fold to about five-fold, about one-fold to about 10-fold, about one-fold to about 100-fold, about one-fold to about 1000-fold, about five-fold to about 10-fold, about five-fold to about 100-fold, about five-fold to about 1000-fold, about 10-fold to about 100-fold, about 10-fold to about 1000-fold, about 10-fold to about 10,000-fold, or about 10-fold to about 100,000-fold.

In yet another embodiment, a targeting domain disclosed herein has a disassociation rate constant that confers preferential binding to its cognate receptor. In other aspects of this embodiment, a targeting domain disclosed herein binds to its cognate receptor with a disassociation rate constant of less than 1 x 10⁻³ s⁻¹, less than 1 x 10⁻⁴ s⁻¹, or less than 1 x 10⁻⁵ s⁻¹. In yet other aspects of this embodiment, a targeting domain disclosed herein binds to its cognate receptor with a disassociation rate constant of, *e.g.,* less than 1.0 x 10⁻⁴ s⁻¹, less than 2.0 x 10⁻⁴ s⁻¹, less than 3.0 x 10⁻⁴ s⁻¹, less than 4.0 x 10⁻⁴ s⁻¹, less than 5.0 x 10⁻⁴ s⁻¹, less than 6.0 x 10⁻⁴ s⁻¹, less than 7.0 x 10⁻⁴ s⁻¹, less than 8.0 x 10⁻⁴ s⁻¹, or less than 9.0 x 10⁻⁴ s⁻¹. In still other aspects of this embodiment, a targeting domain disclosed herein binds to its cognate receptor with a disassociation rate constant of, *e.g.,* more than 1 x 10⁻³ s⁻¹, more than 1 x 10⁻⁴ s⁻¹, or more than 1 x 10⁻⁵ s⁻¹. In other aspects of this embodiment, a targeting domain disclosed herein binds to its cognate receptor with a disassociation rate constant of, e.g., more than 1.0 x 10⁻⁴ s⁻¹, more than 2.0 x 10⁻⁴ s⁻¹, more than 3.0 x 10⁻⁴ s⁻¹, more than 4.0 x 10⁻⁴ s⁻¹, more than 5.0 x 10⁻⁴ s⁻¹, more than 6.0 x 10⁻⁴ s⁻¹, more than 7.0 x 10⁻⁴ s⁻¹, more than 8.0 x 10⁻⁴ s⁻¹, or more than 9.0 x 10⁻⁴ s⁻¹.

In still another embodiment, a targeting domain disclosed herein has a disassociation rate constant that is less for its cognate target receptor relative to a non-cognate receptor. In other aspects of this embodiment, a targeting domain disclosed herein has a disassociation rate constant that is less for its cognate target receptor relative to a non-cognate receptor by, e.g., at least one-fold, at least two-fold, at least three-fold, at least four fold, at least five-fold, at least 10 fold, at least 50 fold, at least 100 fold, at least 1000 fold, at least 10,000 fold, or at least 100,000 fold. In other aspects of this embodiment, a targeting domain disclosed herein has a disassociation rate constant that is less for its cognate target receptor relative to a non-cognate receptor by, e.g., about one-fold to about three-fold, about one-fold to about five-fold, about one-fold to about 10-fold, about one-fold to about 100-fold, about one-fold to about 1000-fold, about five-fold to about 10-fold, about five-fold to about 100-fold, about five-fold to about 1000-fold, about 10-fold to about 100-fold, about 10-fold to about 1000-fold, about 10-fold to about 10,000-fold, or about 10-fold to about 100,000-fold.

In another embodiment, a targeting domain disclosed herein has an equilibrium disassociation constant that confers preferential binding to its cognate receptor. In other aspects of this embodiment, a targeting domain disclosed herein binds to its cognate receptor with an equilibrium disassociation constant of, *e.g.,* less than 0.500 nM. In yet other aspects of this embodiment, a targeting domain disclosed herein binds to its cognate receptor with an equilibrium disassociation constant of, e.g., less than 0.500 nM, less than 0.450 nM, less than 0.400 nM, less than 0.350 nM, less than 0.300 nM, less than 0.250 nM, less than 0.200 nM, less than 0.150 nM, less than 0.100 nM, or less than 0.050 nM. In other aspects of this embodiment, a targeting domain disclosed herein binds to its cognate receptor with an equilibrium disassociation constant of, *e.g.,* more than 0.500 nM, more than 0.450 nM, more than 0.400 nM, more than 0.350 nM, more than 0.300 nM, more than 0.250 nM, more than 0.200 nM, more than 0.150 nM, more than 0.100 nM, or more than 0.050 nM.

In yet another embodiment, a targeting domain disclosed herein has an equilibrium disassociation constant that is greater for its cognate target receptor relative to a non-cognate receptor. In other aspects of this embodiment, a targeting domain disclosed herein has an equilibrium disassociation constant that is greater for its cognate target receptor relative to a non-cognate receptor by, *e.g.,* at least one-fold, at least two-fold, at least three-fold, at least four fold, at least five-fold, at least 10 fold, at least 50 fold, at least 100 fold, at least 1000 fold, at least 10,000 fold, or at least 100,000 fold. In other aspects of this embodiment, a targeting domain disclosed herein has an equilibrium disassociation constant that is greater for its cognate target receptor relative to a non-cognate receptor by, *e.g.,* about one-fold to about three-fold, about one-fold to about five-fold, about one-fold to about 10-fold, about one-fold to about 100-fold, about one-fold to about 1000-fold, about five-fold to about 10-fold, about five-fold to about 100-fold, about five-fold to about 1000-fold, about 10-fold to about 100-fold, about 10-fold to about 1000-fold, about 10-fold to about 10,000-fold, or about 10-fold to about 100,000-fold.

In another embodiment, a targeting domain disclosed herein may be one that preferentially interacts with a receptor located on a sensory neuron. In an aspect of this embodiment, the sensory neuron targeting domain is one whose cognate receptor is located exclusively on the plasma membrane of sensory neurons. In another aspect of this embodiment, the sensory neuron targeting domain is one whose cognate receptor is located primarily on the plasma membrane of sensory neuron. For example, a receptor for a sensory neuron targeting domain is located primarily on a sensory neuron when, e.g., at least 60% of all cells that have a cognate receptor for a sensory neuron targeting domain on the surface of the plasma membrane are sensory neurons, at least 70% of all cells that have a cognate receptor for a sensory neuron targeting domain on the surface of the plasma membrane are sensory neurons, at least 80% of all cells that have a cognate receptor for a sensory neuron targeting domain on the surface of the plasma membrane are sensory neurons, or at least 90% of all cells that have a cognate receptor for a sensory neuron targeting domain on the surface of the plasma membrane are sensory neurons. In yet another aspect of this embodiment, the sensory neuron targeting domain is one whose cognate receptor is located on the plasma membrane of several types of cells, including sensory neurons. In still another aspect of this embodiment, the sensory neuron targeting domain is one whose cognate receptor is located on the plasma membrane of several types of cells, including sensory neurons, with the proviso that motor neurons are not one of the other types of cells.

In another embodiment, a targeting domain disclosed herein may be one that preferentially interacts with a receptor located on a sympathetic neuron. In an aspect of this embodiment, the sympathetic neuron targeting domain is one whose cognate receptor is located exclusively on the plasma membrane of sympathetic neurons. In another aspect of this embodiment, the sympathetic neuron targeting domain is one whose cognate receptor is located primarily on the plasma membrane of sympathetic neuron. For example, a receptor for a sympathetic neuron targeting domain is located primarily on a sympathetic neuron when, *e.g.,* at least 60% of all cells that have a cognate receptor for a sympathetic neuron targeting domain on the surface of the plasma membrane are sympathetic neurons, at least 70% of all cells that have a cognate receptor for a sympathetic neuron targeting domain on the surface of the plasma membrane are sympathetic neurons, at least 80% of all cells that have a cognate receptor for a sympathetic neuron targeting domain on the surface of the plasma membrane are sympathetic neurons, or at least 90% of all cells that have a cognate receptor for a sympathetic neuron targeting domain on the surface of the plasma membrane are sympathetic neurons. In yet another aspect of this embodiment, the sympathetic neuron targeting domain is one whose cognate receptor is located on the plasma membrane of several types of cells, including sympathetic neurons. In still another aspect of this embodiment, the sympathetic neuron targeting domain is one whose cognate receptor is located on the plasma membrane of several types of cells, including sympathetic neurons, with the proviso that motor neurons are not one of the other types of cells.

In another embodiment, a targeting domain disclosed herein may be one that preferentially interacts with a receptor located on a parasympathetic neuron. In an aspect of this embodiment, the parasympathetic neuron targeting domain is one whose cognate receptor is located exclusively on the plasma membrane of parasympathetic neurons. In another aspect of this embodiment, the parasympathetic neuron targeting domain is one whose cognate receptor is located primarily on the plasma membrane of parasympathetic neuron. For example, a receptor for a parasympathetic neuron targeting domain is located primarily on a parasympathetic neuron when, e.g., at least 60% of all cells that have a cognate receptor for a parasympathetic neuron targeting domain on the surface of the plasma membrane are parasympathetic neurons, at least 70% of all cells that have a cognate receptor for a parasympathetic neuron targeting domain on the surface of the plasma membrane are parasympathetic neurons, at least 80% of all cells that have a cognate receptor for a parasympathetic neuron targeting domain on the surface of the plasma membrane are parasympathetic neurons, or at least 90% of all cells that have a cognate receptor for a parasympathetic neuron targeting domain on the surface of the plasma membrane are parasympathetic neurons. In yet another aspect of this embodiment, the parasympathetic neuron targeting domain is one whose cognate receptor is located on the plasma membrane of several types of cells, including parasympathetic neurons. In still another aspect of this embodiment, the parasympathetic neuron targeting domain is one whose cognate receptor is located on the plasma membrane of several types of cells, including parasympathetic neurons, with the proviso that motor neurons are not one of the other types of cells.

In another embodiment, a targeting domain disclosed herein is an opioid peptide targeting domain, a galanin peptide targeting domain, a PAR peptide targeting domain, a somatostatin peptide targeting domain, a neurotensin peptide targeting domain, a SLURP peptide targeting domain, an angiotensin peptide targeting domain, a tachykinin peptide targeting domain, a Neuropeptide Y related peptide targeting domain, a kinin peptide targeting domain, a melanocortin peptide targeting domain, or a granin peptide targeting domain, a glucagon like hormone peptide targeting domain, a secretin peptide targeting domain, a pituitary adenylate cyclase activating peptide (PACAP) peptide targeting domain, a growth hormone-releasing hormone (GHRH) peptide targeting domain, a vasoactive intestinal peptide (VIP) peptide targeting domain, a gastric inhibitory peptide (GIP) peptide targeting domain, a calcitonin peptide targeting domain, a visceral gut peptide targeting domain, a neurotrophin peptide targeting domain, a head activator (HA) peptide, a glial cell line-derived neurotrophic factor (GDNF) family of ligands (GFL) peptide targeting domain, a RF-amide related peptide (RFRP) peptide targeting domain, a neurohormone peptide targeting domain, or a neuroregulatory cytokine peptide targeting domain, an interleukin (IL) targeting domain, vascular endothelial growth factor (VEGF) targeting domain, an insulin-like growth factor (IGF) targeting domain, an epidermal growth factor (EGF) targeting domain, a Transformation Growth Factor-β (TGFβ) targeting domain, a Bone Morphogenetic Protein (BMP) targeting domain, a Growth and Differentiation Factor (GDF) targeting domain, an activin targeting domain, or a Fibroblast Growth Factor (FGF) targeting domain, or a Platelet-Derived Growth Factor (PDGF) targeting domain.

In an aspect of this embodiment, an opioid peptide targeting domain is an enkephalin peptide, a bovine adrenomedullary-22 (BAM22) peptide, an endomorphin peptide, an endorphin peptide, a dynorphin peptide, a nociceptin peptide, or a hemorphin peptide. In another aspect of this embodiment, an enkephalin peptide targeting domain is a Leu-enkephalin peptide, a Met-enkephalin peptide, a Met-enkephalin MRGL peptide, or a Met-enkephalin MRF peptide. In another aspect of this embodiment, a bovine adrenomedullary-22 peptide targeting domain is a BAM22 (1-12) peptide, a BAM22 (6-22) peptide, a BAM22 (8-22) peptide, or a BAM22 (1-22) peptide. In another aspect of this embodiment, an endomorphin peptide targeting domain is an endomorphin-1 peptide or an endomorphin-2 peptide. In another aspect of this embodiment, an endorphin peptide targeting domain an endorphin-α peptide, a neoendorphin-α peptide, an endorphin-β peptide, a neoendorphin-β peptide, or an endorphin-γ peptide. In another aspect of this embodiment, a dynorphin peptide targeting domain is a dynorphin A peptide, a dynorphin B (leumorphin) peptide, or a rimorphin peptide. In another aspect of this embodiment, a nociceptin peptide targeting domain is a nociceptin RK peptide, a nociceptin peptide, a neuropeptide 1 peptide, a neuropeptide 2 peptide, or a neuropeptide 3 peptide. In another aspect of this embodiment, a hemorphin peptide targeting domain is a LVVH7 peptide, a VVH7 peptide, a VH7 peptide, a H7 peptide, a LWH6 peptide, a LWH5 peptide, a WH5 peptide, a LVVH4 peptide, or a LWH3 peptide.

In an aspect of this embodiment, a galanin peptide targeting domain is a galanin peptide, a galanin message-associated peptide (GMAP) peptide, a galanin like protein (GALP) peptide, or an alarin peptide.

In an aspect of this embodiment, a PAR peptide targeting domain is a PAR1 peptide, a PAR2 peptide, a PAR3 peptide and a PAR4 peptide. In an aspect of this embodiment, a somatostatin peptide targeting domain is a somatostatin peptide or a cortistatin peptide. In an aspect of this embodiment, a neurotensin peptide targeting domain a neurotensin or a neuromedin N. In an aspect of this embodiment, a SLURP peptide targeting domain is a SLURP-1 peptide or a SLURP-2 peptide. In an aspect of this embodiment, an angiotensin peptide targeting domain is an angiotensin peptide.

In an aspect of this embodiment, a tachykinin peptide targeting domain is a Substance P peptide, a neuropeptide K peptide, a neuropeptide gamma peptide, a neurokinin A peptide, a neurokinin B peptide, a hemokinin peptide, or a endokinin peptide. In an aspect of this embodiment, a Neuropeptide Y related peptide targeting domain is a Neuropeptide Y peptide, a Peptide YY peptide, Pancreatic peptide peptide, a Pancreatic icosapeptide peptide, a Pancreatic Hormone domain peptide, a CXCL12 peptide, and a Sjogren syndrome antigen B peptide. In an aspect of this embodiment, a kinin peptide targeting domain is a bradykinin peptide, a kallidin peptide, a desArg9 bradykinin peptide, a desArg10 bradykinin peptide, a kininogen peptide, gonadotropin releasing hormone 1 peptide, chemokine peptide, an arginine vasopressin peptide.

In an aspect of this embodiment, a melanocortin peptide targeting domain comprises a melanocyte stimulating hormone peptide, an adrenocorticotropin peptide, a lipotropin peptide, or a melanocortin peptide derived neuropeptide. In an aspect of this embodiment, a melanocyte stimulating hormone peptide targeting domain comprises an α-melanocyte stimulating hormone peptide, a β-melanocyte stimulating hormone peptide, or a γ-melanocyte stimulating hormone peptide. In an aspect of this embodiment, an adrenocorticotropin peptide targeting domain comprises an adrenocorticotropin or a Corticotropin-like intermediary peptide. In an aspect of this embodiment, a lipotropin peptide targeting domain comprises a β-lipotropin peptide or a γ-lipotropin peptide.

In an aspect of this embodiment, a granin peptide targeting domain comprises a chromogranin A peptide, a chromogranin B peptide, a chromogranin C (secretogranin II) peptide, a secretogranin IV peptide, or a secretogranin VI peptide. In an aspect of this embodiment, a chromogranin A peptide targeting domain comprises a β-granin peptide, a vasostatin peptide, a chromostatin peptide, a pancreastatin peptide, a WE-14 peptide, a catestatin peptide, a parastatin peptide, or a GE-25 peptide. In an aspect of this embodiment, a chromogranin B peptide targeting domain comprises a GAWK peptide, an adrenomedullary peptide, or a secretolytin peptide. In an aspect of this embodiment, a chromogranin C peptide targeting domain comprises a secretoneurin peptide.

In an aspect of this embodiment, a glucagons-like hormone peptide targeting domain is a glucagon-like peptide -1, a glucagon-like peptide-2, a glicentin, a glicentin-related peptide (GRPP), a glucagon, or an oxyntomodulin (OXY). In an aspect of this embodiment, a secretin peptide targeting domain is a secretin peptide. In an aspect of this embodiment, a pituitary adenylate cyclase activating peptide targeting domain is a pituitary adenylate cyclase activating peptide. In an aspect of this embodiment, a growth hormone-releasing hormone peptide targeting domain a growth hormone-releasing hormone peptide. In an aspect of this embodiment, a vasoactive intestinal peptide targeting domain is a vasoactive intestinal peptide-1 peptide or a vasoactive intestinal peptide-2 peptide. In an aspect of this embodiment, a gastric inhibitory peptide targeting domain is a gastric inhibitory peptide. In an aspect of this embodiment, a calcitonin peptide targeting domain is a calcitonin peptide, an amylin peptide, a calcitonin-related peptide α, a calcitonin-related peptide β, and a islet amyloid peptide. In an aspect of this embodiment, a visceral gut peptide targeting domain is a gastrin peptide, a gastrin-releasing peptide, or a cholecystokinin peptide.

In an aspect of this embodiment, a neurotrophin peptide targeting domain is a nerve growth factor (NGF) peptide, a brain derived neurotrophic factor (BDNF) peptide, a neurotrophin-3 (NT-3) peptide, a neurotrophin-4/5 (NT-4/5) peptide, or an amyloid beta (A4) precursor protein neurotrophin (APP) peptide. In an aspect of this embodiment, a head activator peptide targeting domain is a head activator peptide. In an aspect of this embodiment, a glial cell line-derived neurotrophic factor family of ligands peptide targeting domain is a glial cell line-derived neurotrophic factor peptide, a Neurturin peptide, a Persephrin peptide, or an Artemin peptide. In an aspect of this embodiment, a RF-amide related peptide targeting domain a RF-amide related peptide-1, a RF-amide related peptide-2, a RF-amide related peptide-3, a neuropeptide AF, or a neuropeptide FF.

In an aspect of this embodiment, a neurohormone peptide targeting domain is a corticotropin-releasing hormone (CCRH), a parathyroid hormone (PTH), a parathyroid hormone-like hormone (PTHLH), a PHYH, a thyrotropin-releasing hormone (TRH), an urocortin-1 (UCN1), an urocortin-2

(UCN2), an urocortin-3 (UCN3), or an urotensin 2 (UTS2). In an aspect of this embodiment, a neuroregulatory cytokine peptide targeting domain is a ciliary neurotrophic factor peptide, a glycophorin-A peptide, a leukemia inhibitory factor peptide, a cardiotrophin-1 peptide, a cardiotrophin-like cytokine peptide, a neuroleukin peptide, and an onostatin M peptide. In an aspect of this embodiment, an IL peptide targeting domain is an IL-1 peptide, an IL-2 peptide, an IL-3 peptide, an IL-4 peptide, an IL-5 peptide, an IL-6 peptide, an IL-7 peptide, an IL-8 peptide, an IL-9 peptide, an IL-10 peptide, an IL-11 peptide, an IL-12 peptide, an IL-18 peptide, an IL-32 peptide, or an IL-33 peptide.

In an aspect of this embodiment, a VEGF peptide targeting domain is a VEGF-A peptide, a VEGF-B peptide, a VEGF-C peptide, a VEGF-D peptide, or a placenta growth factor (PIGF) peptide. In an aspect of this embodiment, an IGF peptide targeting domain is an IGF-1 peptide or an IGF-2 peptide. In an aspect of this embodiment, an EGF peptide targeting domain an EGF, a heparin-binding EGF-like growth factor (HB-EGF), a transforming growth factor-α (TGF-α), an amphiregulin (AR), an epiregulin (EPR), an epigen (EPG), a betacellulin (BTC), a neuregulin-1 (NRG1), a neuregulin-2 (NRG2), a neuregulin-3, (NRG3), or a neuregulin-4 (NRG4). In an aspect of this embodiment, a FGF peptide targeting domain is a FGF1 peptide, a FGF2 peptide, a FGF3 peptide, a FGF4 peptide, a FGF5 peptide, a FGF6 peptide, a FGF7 peptide, a FGF8 peptide, a FGF9 peptide, a FGF10 peptide, a FGF17 peptide, or a FGF18 peptide. In an aspect of this embodiment, a PDGF peptide targeting domain is a PDGFα peptide or a PDGFβ peptide.

In an aspect of this embodiment, a TGFβ peptide targeting domain is a TGFβ1 peptide, a TGFβ2 peptide, a TGFβ3 peptide, or a TGFβ4 peptide. In an aspect of this embodiment, a BMP peptide targeting domain is a BMP2 peptide, a BMP3 peptide, a BMP4 peptide, a BMP5 peptide, a BMP6 peptide, a BMP7 peptide, a BMP8 peptide, or a BMP10 peptide. In an aspect of this embodiment, a GDF peptide targeting domain is a GDF1 peptide, a GDF2 peptide, a GDF3 peptide, a GDF5 peptide, a GDF6 peptide, a GDF7 peptide, a GDF8 peptide, a GDF10 peptide, a GDF11 peptide, or a GDF15 peptide. In an aspect of this embodiment, an activin peptide targeting domain is an activin A peptide, an activin B peptide, an activin C peptide, an activin E peptide, or an inhibin A peptide.

As discussed above, naturally-occurring Clostridial toxins are organized into three functional domains comprising a linear amino-to-carboxyl single polypeptide order of the enzymatic domain (amino region position), the translocation domain (middle region position) and the binding domain (carboxyl region position). This naturally-occurring order can be referred to as the carboxyl presentation of the binding domain because the domain necessary for binding to the receptor is located at the carboxyl region position of the Clostridial toxin. However, it has been shown that Clostridial toxins can be modified by rearranging the linear amino-to-carboxyl single polypeptide order of the three major domains and locating a targeting moiety at the amino region position of a Clostridial toxin, referred to as amino presentation, as well as in the middle region position, referred to as central presentation.

Thus, a TEM can comprise a targeting domain in any and all locations with the proviso that TEM is capable of performing the intoxication process. Non-limiting examples include, locating a targeting domain at the amino terminus of a TEM; locating a targeting domain between a Clostridial toxin enzymatic domain and a Clostridial toxin translocation domain of a TEM; and locating a targeting domain at the carboxyl terminus of a TEM. Other non-limiting examples include, locating a targeting domain between a Clostridial toxin enzymatic domain and a Clostridial toxin translocation domain of a TEM. The enzymatic domain of naturally-occurring Clostridial toxins contains the native start methionine. Thus, in domain organizations where the enzymatic domain is not in the amino-terminal location an amino acid sequence comprising the start methionine should be placed in front of the amino-terminal domain. Likewise, where a targeting domain is in the amino-terminal position, an amino acid sequence comprising a start methionine and a protease cleavage site may be operably-linked in situations in which a targeting domain requires a free amino terminus, see, e.g., Shengwen Li et al., *Degradable Clostridial Toxins,* U.S. Patent Application 11/572,512 (Jan. 23, 2007), entirely incorporated by reference. in its entirety. In addition, it is known in the art that when adding a polypeptide that is operably-linked to the amino terminus of another polypeptide comprising the start methionine that the original methionine residue can be deleted.

A TEM disclosed herein may optionally comprise an exogenous protease cleavage site that allows the use of an exogenous protease to convert the single-chain polypeptide form of a TEM into its more active di-chain form. As used herein, the term "exogenous protease cleavage site" is synonymous with a "non-naturally occurring protease cleavage site" or "non-native protease cleavage site" and means a protease cleavage site that is not naturally found in a di-chain loop region from a naturally occurring Clostridial toxin.

Naturally-occurring Clostridial toxins are each translated as a single-chain polypeptide of approximately 150 kDa that is subsequently cleaved by proteolytic scission within a disulfide loop by a naturally-occurring protease. This cleavage occurs within the discrete di-chain loop region located between two cysteine residues that form a disulfide bridge and comprising an endogenous protease cleavage site. As used herein, the term "endogenous di-chain loop protease cleavage site" is synonymous with a "naturally occurring di-chain loop protease cleavage site" and refers to a naturally occurring protease cleavage site found within the di-chain loop region of a naturally occurring Clostridial toxin. This posttranslational processing yields a di-chain molecule comprising an approximately 50 kDa light chain, comprising the enzymatic domain, and an approximately 100 kDa heavy chain, comprising the translocation and cell binding domains, the light chain and heavy chain being held together by the single disulfide bond and non-covalent interactions. Recombinantly-produced Clostridial toxins generally substitute the naturally-occurring di-chain loop protease cleavage site with an exogenous protease cleavage site to facilitate production of a recombinant di-chain molecule. See e.g., Dolly, J.O. et al., *Activatable Clostridial Toxins,* U.S. Patent No. 7,419,676 (Sep. 2, 2008), entirely incorporated by reference.

Although TEMs vary in their overall molecular weight because the size of the targeting domain, the activation process and its reliance on an exogenous cleavage site is essentially the same as that for recombinantly-produced Clostridial toxins. *See e.g.,* Steward, et al., *Activatable Clostridial Toxins,* US 2009/0081730; Steward, et al., *Modified Clostridial Toxins with Enhanced Translocation Capabilities and Altered Targeting Activity For Non-Clostridial Toxin Target Cells,* U.S. Patent Application No. 11/776,075; Steward, et al., *Modified Clostridial Toxins with Enhanced Translocation Capabilities and Altered Targeting Activity for Clostridial Toxin Target Cells,* US 2008/0241881, each entirely incorporated by reference. In general, the activation process that converts the single-chain polypeptide into its di-chain form using exogenous proteases can be used to process TEMs having a targeting domain organized in an amino presentation, central presentation, or carboxyl presentation arrangement. This is because for most targeting domains the amino-terminus of the moiety does not participate in receptor binding. As such, a wide range of protease cleavage sites can be used to produce an active di-chain form of a TEM. However, targeting domains requiring a free amino-terminus for receptor binding require a protease cleavage site whose scissile bond is located at the carboxyl terminus. The use of protease cleavage site is the design of a TEM are described in, *e.g.,* Steward, et al., Activatable Clostridial toxins, US 2009/0069238; Ghanshani, et al., Modified Clostridial Toxins Comprising an Integrated Protease Cleavage Site-Binding Domain, US 2011/0189162; and Ghanshani, et al., Methods of Intracellular Conversion of Single-Chain Proteins into their Di-chain Form, International Patent Application Serial No. PCT/US2011/22272, each entirely incorporated by reference.

Non-limiting examples of exogenous protease cleavage sites include, e.g., a plant papain cleavage site, an insect papain cleavage site, a crustacian papain cleavage site, an enterokinase protease cleavage site, a Tobacco Etch Virus protease cleavage site, a Tobacco Vein Mottling Virus protease cleavage site, a human rhinovirus 3C protease cleavage site, a human enterovirus 3C protease cleavage site, a subtilisin cleavage site, a hydroxylamine cleavage site, a SUMO/ULP-1 protease cleavage site, and a Caspase 3 cleavage site.

Thus, in an embodiment, a TEM can comprise an amino to carboxyl single polypeptide linear order comprising a targeting domain, a translocation domain, an exogenous protease cleavage site and an enzymatic domain. In an aspect of this embodiment, a TEM can comprise an amino to carboxyl single polypeptide linear order comprising a targeting domain, a Clostridial toxin translocation domain, an exogenous protease cleavage site and a Clostridial toxin enzymatic domain.

In another embodiment, a TEM can comprise an amino to carboxyl single polypeptide linear order comprising a targeting domain, an enzymatic domain, an exogenous protease cleavage site, and a translocation domain. In an aspect of this embodiment, a TEM can comprise an amino to carboxyl single polypeptide linear order comprising a targeting domain, a Clostridial toxin enzymatic domain, an exogenous protease cleavage site, a Clostridial toxin translocation domain.

In yet another embodiment, a TEM can comprise an amino to carboxyl single polypeptide linear order comprising an enzymatic domain, an exogenous protease cleavage site, a targeting domain, and a translocation domain. In an aspect of this embodiment, a TEM can comprise an amino to carboxyl single polypeptide linear order comprising a Clostridial toxin enzymatic domain, an exogenous protease cleavage site, a targeting domain, and a Clostridial toxin translocation domain.

In yet another embodiment, a TEM can comprise an amino to carboxyl single polypeptide linear order comprising a translocation domain, an exogenous protease cleavage site, a targeting domain, and an enzymatic domain. In an aspect of this embodiment, a TEM can comprise an amino to carboxyl single polypeptide linear order comprising a Clostridial toxin translocation domain, a targeting domain, an exogenous protease cleavage site and a Clostridial toxin enzymatic domain.

In another embodiment, a TEM can comprise an amino to carboxyl single polypeptide linear order comprising an enzymatic domain, a targeting domain, an exogenous protease cleavage site, and a translocation domain. In an aspect of this embodiment, a TEM can comprise an amino to carboxyl single polypeptide linear order comprising a Clostridial toxin enzymatic domain, a targeting domain, an exogenous protease cleavage site, a Clostridial toxin translocation domain.

In yet another embodiment, a TEM can comprise an amino to carboxyl single polypeptide linear order comprising a translocation domain, a targeting domain, an exogenous protease cleavage site and an enzymatic domain. In an aspect of this embodiment, a TEM can comprise an amino to carboxyl single polypeptide linear order comprising a Clostridial toxin translocation domain, a targeting domain, an exogenous protease cleavage site and a Clostridial toxin enzymatic domain.

In still another embodiment, a TEM can comprise an amino to carboxyl single polypeptide linear order comprising an enzymatic domain, an exogenous protease cleavage site, a translocation domain, and a targeting domain. In an aspect of this embodiment, a TEM can comprise an amino to carboxyl single polypeptide linear order comprising a Clostridial toxin enzymatic domain, an exogenous protease cleavage site, a Clostridial toxin translocation domain, and a targeting domain.

In still another embodiment, a TEM can comprise an amino to carboxyl single polypeptide linear order comprising a translocation domain, an exogenous protease cleavage site, an enzymatic domain and a targeting domain. In an aspect of this embodiment, a TEM can comprise an amino to carboxyl single polypeptide linear order comprising a Clostridial toxin translocation domain, a targeting domain, an exogenous protease cleavage site and a Clostridial toxin enzymatic domain.

Non-limiting examples of TEMs disclosed herein, including TEMs comprising a Clostridal toxin enzymatic domain, a Clostridial toxin translocation domain and a targeting domain, the use of an exogenous protease cleavage site, and the design of amino presentation, central presentation and carboxyl presentation TEMs are described in, *e.g.,* US 7,959,933, Activatable Recombinant Neurotoxins, US 7,897,157, Activatable Clostridial Toxins; US 7,833,535, Clostridial Toxin Derivatives and Methods for Treating Pain; US 7,811,584, Multivalent Clostridial Toxins; US 7,780,968, Clostridial Toxin Derivatives and Methods for Treating Pain; US 7,749,514, Activatable Clostridial Toxins, US 7,740,868, Activatable Clostridial Toxins; US 7,736,659, Clostridial Toxin Derivatives and Methods for Treating Pain; US 7,709,228, Activatable Recombinant Neurotoxins; US 7,704,512, Clostridial Toxin Derivatives and Methods for Treating Pain; US 7,659,092, Fusion Proteins; US 7,658,933, Non-Cytotoxic Protein Conjugates; US 7,622,127, Clostridial Toxin Derivatives and Methods for Treating Pain; US 7,514,088, Multivalent Clostridial Toxin Derivatives and Methods of Their Use; US 7,425,338, Clostridial Toxin Derivatives and Methods for Treating Pain; US 7,422,877, Activatable Recombinant Neurotoxins; US 7,419,676, Activatable Recombinant Neurotoxins; US 7,413,742, Clostridial Toxin Derivatives and Methods for Treating Pain; US 7,262,291, Clostridial Toxin Derivatives and Methods for Treating Pain; US 7,244,437, Clostridial Toxin Derivatives and Methods for Treating Pain; US 7,244,436, Clostridial Toxin Derivatives and Methods for Treating Pain; US 7,138,127, Clostridial Toxin Derivatives and Methods for Treating Pain; US 7,132,259, Activatable Recombinant Neurotoxins; US 7,056,729, Botulinum Neurotoxin-Substance P Conjugate or Fusion Protein for Treating Pain; US 6,641,820, Clostridial Toxin Derivatives and Methods to Treat Pain; US 6,500,436, Clostridial Toxin Derivatives and Methods for Treating Pain; US 2011/0091437, Fusion Proteins; US 2011/0070621, Multivalent Clostridial Toxins; US 2011/0027256, Fusion Proteins; US 2010/0247509, Fusion Proteins; US 2010/0041098, Modified Clostridial Toxins with Altered Targeting Capabilities for Clostridial Toxin Target Cells; US 2010/0034802, Treatment of Pain; US 2009/0162341, Non-Cytotoxic Protein Conjugates; US 2009/0087458, Activatable Recombinant Neurotoxins; US 2009/0081730, Activatable Recombinant Neurotoxins; US 2009/0069238, Activatable Clostridial Toxins; US 2009/0042270, Activatable Recombinant Neurotoxins; US 2009/0030182, Activatable Recombinant Neurotoxins; US 2009/0018081, Activatable Clostridial Toxins; US 2009/0005313, Activatable Clostridial Toxins; US 2009/0004224, Activatable Clostridial Toxins; US 2008/0317783, Clostridial Toxin Derivatives and Methods for Treating Pain; US 2008/0241881, Modified Clostridial Toxins with Enhanced Translocation Capabilities and Altered Targeting Activity for Clostridial Toxin Target Cells; WO 2006/099590, Modified Clostridial Toxins with Altered Targeting Capabilities for Clostridial Toxin Target Cells; WO 2006/101809, Modified Clostridial Toxins with Enhanced Targeting Capabilities for Endogenous Clostridial Toxin Receptor Systems; WO 2007/106115, Modified Clostridial Toxins with Altered Targeting Capabilities for Clostridial Toxin Target Cells; WO 2008/008803, Modified Clostridial Toxins with Enhanced Translocation Capabilities and Altered Targeting Activity for Clostridial Toxin Target Cells; WO 2008/008805, Modified Clostridial Toxins with Enhanced Translocation Capabilities and Altered Targeting Activity For Non-Clostridial Toxin Target Cells; WO 2008/105901, Modified Clostridial Toxins with Enhanced Translocation Capability and Enhanced Targeting Activity; WO 2011/020052, Methods of Treating Cancer Using Opioid Retargeted Endpeptidases; WO 2011/020056, Methods of Treating Cancer Using Galanin Retargeted Endpeptidases; WO 2011/020114, Methods of Treating Cancer Using Tachykinin Retargeted Endopeptidases; WO 2011/020115, Methods of Treating Cancer Using Growth Factor Retargeted Endopeptidases; WO 2011/020117, Methods of Treating Cancer Using Neurotrophin Retargeted Endopeptidases; WO 2011/020119, Methods of Treating Cancer Using Glucagon-Like Hormone Retargeted Endopeptidases; each entirely incorporated by reference.

A composition disclosed herein is generally administered as a pharmaceutical acceptable composition comprising a TEM. As used herein, the term "pharmaceutically acceptable" means any molecular entity or composition that does not produce an adverse, allergic or other untoward or unwanted reaction when administered to an individual. As used herein, the term "pharmaceutically acceptable composition" is synonymous with "pharmaceutical composition" and means a therapeutically effective concentration of an active ingredient, such as, e.g., any of the TEMs disclosed herein. A pharmaceutical composition comprising a TEM is useful for medical and veterinary applications. A pharmaceutical composition may be administered to an individual alone, or in combination with other supplementary active ingredients, agents, drugs or hormones. The pharmaceutical compositions may be manufactured using any of a variety of processes, including, without limitation, conventional mixing, dissolving, granulating, dragee-making, levigating, emulsifying, encapsulating, entrapping, and lyophilizing. The pharmaceutical composition can take any of a variety of forms including, without limitation, a sterile solution, suspension, emulsion, lyophilizate, tablet, pill, pellet, capsule, powder, syrup, elixir or any other dosage form suitable for administration.

Aspects of the present specification provide, in part, a composition comprising a TEM. It is envisioned that any of the composition disclosed herein can be useful in a method of treating disclosed herein, with the proviso that the composition prevents or reduces a symptom associated with condition being treated. It is also understood that the two or more different TEMs can be provided as separate compositions or as part of a single composition.

A pharmaceutical composition comprising a TEM may optionally include a pharmaceutically acceptable carrier that facilitates processing of an active ingredient into pharmaceutically acceptable compositions. As used herein, the term "pharmacologically acceptable carrier" is synonymous with "pharmacological carrier" and means any carrier that has substantially no long term or permanent detrimental effect when administered and encompasses terms such as "pharmacologically acceptable vehicle, stabilizer, diluent, additive, auxiliary or excipient." Such a carrier generally is mixed with an active compound, or permitted to dilute or enclose the active compound and can be a solid, semi-solid, or liquid agent. It is understood that the active ingredients can be soluble or can be delivered as a suspension in the desired carrier or diluent. Any of a variety of pharmaceutically acceptable carriers can be used including, without limitation, aqueous media such as, e.g., water, saline, glycine, hyaluronic acid and the like; solid carriers such as, e.g., mannitol, lactose, starch, magnesium stearate, sodium saccharin, talcum, cellulose, glucose, sucrose, magnesium carbonate, and the like; solvents; dispersion media; coatings; antibacterial and antifungal agents; isotonic and absorption delaying agents; or any other inactive ingredient. Selection of a pharmacologically acceptable carrier can depend on the mode of administration. Except insofar as any pharmacologically acceptable carrier is incompatible with the active ingredient, its use in pharmaceutically acceptable compositions is contemplated. Non-limiting examples of specific uses of such pharmaceutical carriers can be found in PHARMACEUTICAL DOSAGE FORMS AND DRUG DELIVERY SYSTEMS (Howard C. Ansel et al., eds., Lippincott Williams & Wilkins Publishers, 7th ed. 1999); REMINGTON: THE SCIENCE AND PRACTICE OF PHARMACY (Alfonso R. Gennaro ed., Lippincott, Williams & Wilkins,, 20th ed. 2000); GOODMAN & GILMAN'S THE PHARMACOLOGICAL BASIS OF THERAPEUTICS (Joel G. Hardman et al., eds., McGraw-Hill Professional, 10th ed. 2001); and HANDBOOK OF PHARMACEUTICAL EXCIPIENTS (Raymond C. Rowe et al., APhA Publications, 4th edition 2003). These protocols are routine procedures and any modifications are well within the scope of one skilled in the art and from the teaching herein.

It is further envisioned that a pharmaceutical composition disclosed herein can optionally include, without limitation, other pharmaceutically acceptable components (or pharmaceutical components), including, without limitation, buffers, preservatives, tonicity adjusters, salts, antioxidants, osmolality adjusting agents, physiological substances, pharmacological substances, bulking agents, emulsifying agents, wetting agents, sweetening or flavoring agents, and the like. Various buffers and means for adjusting pH can be used to prepare a pharmaceutical composition disclosed herein, provided that the resulting preparation is pharmaceutically acceptable. Such buffers include, without limitation, acetate buffers, citrate buffers, phosphate buffers, neutral buffered saline, phosphate buffered saline and borate buffers. It is understood that acids or bases can be used to adjust the pH of a composition as needed. Pharmaceutically acceptable antioxidants include, without limitation, sodium metabisulfite, sodium thiosulfate, acetylcysteine, butylated hydroxyanisole and butylated hydroxytoluene. Useful preservatives include, without limitation, benzalkonium chloride, chlorobutanol, thimerosal, phenylmercuric acetate, phenylmercuric nitrate, a stabilized oxy chloro composition and chelants, such as, e.g., DTPA or DTPA-bisamide, calcium DTPA, and CaNaDTPA-bisamide. Tonicity adjustors useful in a pharmaceutical composition include, without limitation, salts such as, e.g., sodium chloride, potassium chloride, mannitol or glycerin and other pharmaceutically acceptable tonicity adjustor. The pharmaceutical composition may be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. It is understood that these and other substances known in the art of pharmacology can be included in a pharmaceutical composition. Exemplary pharmaceutical composition comprising a TEM are described in Hunt, et al., Animal Protein-Free Pharmaceutical Compositions, US Serial No. 12/331,816; and Dasari, et al., Clostridial Toxin Pharmaceutical Compositions, WO/2010/090677, each entirely incorporated by reference. in its entirety.

In an embodiment, a composition comprising a TEM is a pharmaceutical composition comprising a TEM. In aspects of this embodiment, a pharmaceutical composition comprising a TEM further comprises a pharmacological carrier, a pharmaceutical component, or both a pharmacological carrier and a pharmaceutical component. In other aspects of this embodiment, a pharmaceutical composition comprising a TEM further comprises at least one pharmacological carrier, at least one pharmaceutical component, or at least one pharmacological carrier and at least one pharmaceutical component.

Aspects of the present specification disclose, in part, treating an individual suffering from a migraine disorder. As used herein, the term "treating," refers to reducing or eliminating in an individual a clinical symptom of a migraine disorder; or delaying or preventing in an individual the onset of a clinical symptom of a migraine disorder. For example, the term "treating" can mean reducing a symptom of a condition characterized by a migraine disorder by, e.g., at least 20%, at least 30%, at least 40%, at least 50%, at least 60%, at least 70%, at least 80%, at least 90% or at least 100%. The actual symptoms associated with a migraine disorder are well known and can be determined by a person of ordinary skill in the art by taking into account factors, including, without limitation, the location of the migraine disorder, the cause of the migraine disorder, the severity of the migraine disorder, and/or the tissue or organ affected by the migraine disorder. Those of skill in the art will know the appropriate symptoms or indicators associated with specific migraine disorder and will know how to determine if an individual is a candidate for treatment as disclosed herein.

As used herein, the term "migraine disorder" refers to a migraine disorder where at least one of the underlying symptoms being treated is due to a sensory nerve-based etiology, a sympathetic nerve-based etiology, and/or a parasympathetic nerve-based etiology. Typically such etiologies will involve an abnormal overactivity of a nerve that results in symptoms of a migraine disorder, or any normal activity of a nerve that needs to be reduced or stopped for a period of time in order to treat a migraine disorder. Migraine disorders include, without limitation, a migraine without aura, a migraine with aura, a menstrual migraine, a migraine equivalent, a complicated migraine, an abdominal migraine, or a mixed tension migraine.

A composition or compound is administered to an individual. An individual is typically a human being. Typically, any individual who is a candidate for a conventional migraine disorder treatment is a candidate for a migraine disorder treatment disclosed herein. Pre-operative evaluation typically includes routine history and physical examination in addition to thorough informed consent disclosing all relevant risks and benefits of the procedure.

The amount of a TEM disclosed herein used with the methods of treatment disclosed herein will typically be an effective amount. As used herein, the term "effective amount" is synonymous with "therapeutically effective amount", "effective dose", or "therapeutically effective dose" and when used in reference to treating a migraine disorder means the minimum dose of a TEM necessary to achieve the desired therapeutic effect and includes a dose sufficient to reduce a symptom associated with a migraine disorder. The effectiveness of a TEM disclosed herein in treating a migraine disorder can be determined by observing an improvement in an individual based upon one or more clinical symptoms, and/or physiological indicators associated with the condition. An improvement in a migraine disorder also can be indicated by a reduced need for a concurrent therapy.

The appropriate effective amount of a TEM to be administered to an individual for a particular migraine disorder can be determined by a person of ordinary skill in the art by taking into account factors, including, without limitation, the type of migraine disorder, the location of the migraine disorder, the cause of the migraine disorder, the severity of the migraine disorder, the degree of relief desired, the duration of relief desired, the particular TEM used, the rate of excretion of the TEM used, the pharmacodynamics of the TEM used, the nature of the other compounds to be included in the composition, the particular route of administration, the particular characteristics, history and risk factors of the patient, such as, *e.g.,* age, weight, general health and the like, or any combination thereof. Additionally, where repeated administration of a composition comprising a TEM is used, an effective amount of a TEM will further depend upon factors, including, without limitation, the frequency of administration, the half-life of the composition comprising a TEM, or any combination thereof. In is known by a person of ordinary skill in the art that an effective amount of a composition comprising a TEM can be extrapolated from *in vitro* assays and *in vivo* administration studies using animal models prior to administration to humans.

Wide variations in the necessary effective amount are to be expected in view of the differing efficiencies of the various routes of administration. For instance, oral administration generally would be expected to require higher dosage levels than administration by intravenous or intravitreal injection. Similarly, systemic administration of a TEM would be expected to require higher dosage levels than a local administration. Variations in these dosage levels can be adjusted using standard empirical routines of optimization, which are well-known to a person of ordinary skill in the art. The precise therapeutically effective dosage levels and patterns are preferably determined by the attending physician in consideration of the above-identified factors. One skilled in the art will recognize that the condition of the individual can be monitored throughout the course of therapy and that the effective amount of a TEM disclosed herein that is administered can be adjusted accordingly.

In aspects of this embodiment, a therapeutically effective amount of a composition comprising a TEM reduces a symptom associated with a migraine disorder by, *e.g.,* at least 10%, at least 20%, at least 30%, at least 40%, at least 50%, at least 60%, at least 70%, at least 80%, at least 90% or at least 100%. In other aspects of this embodiment, a therapeutically effective amount of a composition comprising a TEM reduces a symptom associated with a migraine disorder by, *e.g.,* at most 10%, at most 20%, at most 30%, at most 40%, at most 50%, at most 60%, at most 70%, at most 80%, at most 90% or at most 100%. In yet other aspects of this embodiment, a therapeutically effective amount of a composition comprising a TEM reduces a symptom associated with a migraine disorder by, *e.g.,* about 10% to about 100%, about 10% to about 90%, about 10% to about 80%, about 10% to about 70%, about 10% to about 60%, about 10% to about 50%, about 10% to about 40%, about 20% to about 100%, about 20% to about 90%, about 20% to about 80%, about 20% to about 20%, about 20% to about 60%, about 20% to about 50%, about 20% to about 40%, about 30% to about 100%, about 30% to about 90%, about 30% to about 80%, about 30% to about 70%, about 30% to about 60%, or about 30% to about 50%. In still other aspects of this embodiment, a therapeutically effective amount of the TEM is the dosage sufficient to inhibit neuronal activity for, *e.g.,* at least one week, at least one month, at least two months, at least three months, at least four months, at least five months, at least six months, at least seven months, at least eight months, at least nine months, at least ten months, at least eleven months, or at least twelve months.

In other aspects of this embodiment, a therapeutically effective amount of a TEM generally is in the range of about 1 fg to about 3.0 mg. In aspects of this embodiment, an effective amount of a TEM can be, *e.g.,* about 100 fg to about 3.0 mg, about 100 pg to about 3.0 mg, about 100 ng to about 3.0 mg, or about 100 µg to about 3.0 mg. In other aspects of this embodiment, an effective amount of a TEM can be, *e.g.,* about 100 fg to about 750 µg, about 100 pg to about 750 µg, about 100 ng to about 750 µg, or about 1 µg to about 750 µg. In yet other aspects of this embodiment, a therapeutically effective amount of a TEM can be, *e.g.,* at least 1 fg, at least 250 fg, at least 500 fg, at least 750 fg, at least 1 pg, at least 250 pg, at least 500 pg, at least 750 pg, at least 1 ng, at least 250 ng, at least 500 ng, at least 750 ng, at least 1 µg, at least 250 µg, at least 500 µg, at least 750 µg, or at least 1 mg. In still other aspects of this embodiment, a therapeutically effective amount of a composition comprising a TEM can be, e.g., at most 1 fg, at most 250 fg, at most 500 fg, at most 750 fg, at most 1 pg, at most 250 pg, at most 500 pg, at most 750 pg, at most 1 ng, at most 250 ng, at most 500 ng, at most 750 ng, at most 1 µg, at least 250 µg, at most 500 µg, at most 750 µg, or at most 1 mg.

In yet other aspects of this embodiment, a therapeutically effective amount of a TEM generally is in the range of about 0.00001 mg/kg to about 3.0 mg/kg. In aspects of this embodiment, an effective amount of a TEM can be, *e.g.,* about 0.0001 mg/kg to about 0.001 mg/kg, about 0.03 mg/kg to about 3.0 mg/kg, about 0.1 mg/kg to about 3.0 mg/kg, or about 0.3 mg/kg to about 3.0 mg/kg. In yet other aspects of this embodiment, a therapeutically effective amount of a TEM can be, e.g., at least 0.00001 mg/kg, at least 0.0001 mg/kg, at least 0.001 mg/kg, at least 0.01 mg/kg, at least 0.1 mg/kg, or at least 1 mg/kg. In yet other aspects of this embodiment, a therapeutically effective amount of a TEM can be, e.g., at most 0.00001 mg/kg, at most 0.0001 mg/kg, at most 0.001 mg/kg, at most 0.01 mg/kg, at most 0.1 mg/kg, or at most 1 mg/kg.

Dosing can be single dosage or cumulative (serial dosing), and can be readily determined by one skilled in the art. For instance, treatment of a migraine disorder may comprise a one-time administration of an effective dose of a composition comprising a TEM. As a non-limiting example, an effective dose of a composition comprising a TEM can be administered once to an individual, *e.g.,* as a single injection or deposition at or near the site exhibiting a symptom of a migraine disorder. Alternatively, treatment of a migraine disorder may comprise multiple administrations of an effective dose of a composition comprising a TEM carried out over a range of time periods, such as, *e.g.,* daily, once every few days, weekly, monthly or yearly. As a non-limiting example, a composition comprising a TEM can be administered once or twice yearly to an individual. The timing of administration can vary from individual to individual, depending upon such factors as the severity of an individual's symptoms. For example, an effective dose of a composition comprising a TEM can be administered to an individual once a month for an indefinite period of time, or until the patient no longer requires therapy. A person of ordinary skill in the art will recognize that the condition of the individual can be monitored throughout the course of treatment and that the effective amount of a composition comprising a TEM that is administered can be adjusted accordingly.

A composition comprising a TEM can be administered to an individual using a variety of routes. Routes of administration suitable for a method of treating a migraine disorder as disclosed herein include both local and systemic administration. Local administration results in significantly more delivery of a composition to a specific location as compared to the entire body of the individual, whereas, systemic administration results in delivery of a composition to essentially the entire body of the individual. Routes of administration suitable for a method of treating a migraine disorder as disclosed herein also include both central and peripheral administration. Central administration results in delivery of a composition to essentially the central nervous system of an individual and includes, *e.g.,* intrathecal administration, epidural administration as well as a cranial injection or implant. Peripheral administration results in delivery of a composition to essentially any area of an individual outside of the central nervous system and encompasses any route of administration other than direct administration to the spine or brain. The actual route of administration of a composition comprising a TEM used can be determined by a person of ordinary skill in the art by taking into account factors, including, without limitation, the type of migraine disorder, the location of the migraine disorder, the cause of the migraine disorder, the severity of the migraine disorder, the degree of relief desired, the duration of relief desired, the particular TEM used, the rate of excretion of the TEM used, the pharmacodynamics of the TEM used, the nature of the other compounds to be included in the composition, the particular route of administration, the particular characteristics, history and risk factors of the individual, such as, *e.g.,* age, weight, general health and the like, or any combination thereof.

In an embodiment, a composition comprising a TEM is administered systemically to an individual. In another embodiment, a composition comprising a TEM is administered locally to an individual. In an aspect of this embodiment, a composition comprising a TEM is administered to a nerve of an individual. In another aspect of this embodiment, a composition comprising a TEM is administered to the area surrounding a nerve of an individual.

A composition comprising a TEM can be administered to an individual using a variety of delivery mechanisms. The actual delivery mechanism used to administer a composition comprising a TEM to an individual can be determined by a person of ordinary skill in the art by taking into account factors, including, without limitation, the type of migraine disorder, the location of the migraine disorder, the cause of the migraine disorder, the severity of the migraine disorder, the degree of relief desired, the duration of relief desired, the particular TEM used, the rate of excretion of the TEM used, the pharmacodynamics of the TEM used, the nature of the other compounds to be included in the composition, the particular route of administration, the particular characteristics, history and risk factors of the patient, such as, *e.g.,* age, weight, general health and the like, or any combination thereof.

In an embodiment, a composition comprising a TEM is administered by injection. In aspects of this embodiment, administration of a composition comprising a TEM is by, *e.g.,* intramuscular injection, intraorgan injection, subdermal injection, dermal injection, intracranical, spinal, or injection into any other body area for the effective administration of a composition comprising a TEM. In aspects of this embodiment, injection of a composition comprising a TEM is to a nerve or into the area surrounding a nerve.

In another embodiment, a composition comprising a TEM is administered by catheter. In aspects of this embodiment, administration of a composition comprising a TEM is by, *e.g.,* a catheter placed in an epidural space.

A composition comprising a TEM as disclosed herein can also be administered to an individual in combination with other therapeutic compounds to increase the overall therapeutic effect of the treatment. The use of multiple compounds to treat an indication can increase the beneficial effects while reducing the presence of side effects.

Aspects of the present invention can also be described as follows:
1. A method of treating a migraine disorder in a human, the method comprising the step of administering to the human in need thereof a therapeutically effective amount of a composition including a TEM, wherein administration of the composition reduces a symptom of the migraine disorder, thereby treating the human.
2. A use of a TEM in the manufacturing a medicament for treating a migraine disorder in a human in need thereof.
3. A use of a TEM in the treatment of a migraine disorder in a human in need thereof.
4. The embodiments of 1 to 3, wherein the TEM comprises a linear amino-to-carboxyl single polypeptide order of 1) a Clostridial toxin enzymatic domain, a Clostridial toxin translocation domain, a targeting domain, 2) a Clostridial toxin enzymatic domain, a targeting domain, a Clostridial toxin translocation domain, 3) a targeting domain, a Clostridial toxin translocation domain, and a Clostridial toxin enzymatic domain, 4) a targeting domain, a Clostridial toxin enzymatic domain, a Clostridial toxin translocation domain, 5) a Clostridial toxin translocation domain, a Clostridial toxin enzymatic domain and a targeting domain, or 6) a Clostridial toxin translocation domain, a targeting domain and a Clostridial toxin enzymatic domain.
5. The embodiments of 1 to 3, wherein the TEM comprises a linear amino-to-carboxyl single polypeptide order of 1) a Clostridial toxin enzymatic domain, an exogenous protease cleavage site, a Clostridial toxin translocation domain, a targeting domain, 2) a Clostridial toxin enzymatic domain, an exogenous protease cleavage site, a targeting domain, a Clostridial toxin translocation domain, 3) a targeting domain, a Clostridial toxin translocation domain, an exogenous protease cleavage site and a Clostridial toxin enzymatic domain, 4) a targeting domain, a Clostridial toxin enzymatic domain, an exogenous protease cleavage site, a Clostridial toxin translocation domain, 5) a Clostridial toxin translocation domain, an exogenous protease cleavage site, a Clostridial toxin enzymatic domain and a targeting domain, or 6) a Clostridial toxin translocation domain, an exogenous protease cleavage site, a targeting domain and a Clostridial toxin enzymatic domain.
6. The embodiments of 1 to 5, wherein the Clostridial toxin translocation domain is a BoNT/A translocation domain, a BoNT/B translocation domain, a BoNT/C1 translocation domain, a BoNT/D translocation domain, a BoNT/E translocation domain, a BoNT/F translocation domain, a BoNT/G translocation domain, a TeNT translocation domain, a BaNT translocation domain, or a BuNT translocation domain.
7. The embodiments of 1 to 6, wherein the Clostridial toxin enzymatic domain is a BoNT/A enzymatic domain, a BoNT/B enzymatic domain, a BoNT/C1 enzymatic domain, a BoNT/D enzymatic domain, a BoNT/E enzymatic domain, a BoNT/F enzymatic domain, a BoNT/G enzymatic domain, a TeNT enzymatic domain, a BaNT enzymatic domain, or a BuNT enzymatic domain.
8. The embodiments of 1 to 7, wherein the targeting domain is a sensory neuron targeting domain, a sympathetic neuron targeting domain, or a parasympathetic neuron targeting domain.
9. The embodiments of 1 to 7, wherein the targeting domain is an opioid peptide targeting domain, a galanin peptide targeting domain, a PAR peptide targeting domain, a somatostatin peptide targeting domain, a neurotensin peptide targeting domain, a SLURP peptide targeting domain, an angiotensin peptide targeting domain, a tachykinin peptide targeting domain, a Neuropeptide Y related peptide targeting domain, a kinin peptide targeting domain, a melanocortin peptide targeting domain, or a granin peptide targeting domain, a glucagon like hormone peptide targeting domain, a secretin peptide targeting domain, a pituitary adenylate cyclase activating peptide (PACAP) peptide targeting domain, a growth hormone-releasing hormone (GHRH) peptide targeting domain, a vasoactive intestinal peptide (VIP) peptide targeting domain, a gastric inhibitory peptide (GIP) peptide targeting domain, a calcitonin peptide targeting domain, a visceral gut peptide targeting domain, a neurotrophin peptide targeting domain, a head activator (HA) peptide, a glial cell line-derived neurotrophic factor (GDNF) family of ligands (GFL) peptide targeting domain, a RF-amide related peptide (RFRP) peptide targeting domain, a neurohormone peptide targeting domain, or a neuroregulatory cytokine peptide targeting domain, an interleukin (IL) targeting domain, vascular endothelial growth factor (VEGF) targeting domain, an insulin-like growth factor (IGF) targeting domain, an epidermal growth factor (EGF) targeting domain, a Transformation Growth Factor-β (TGFβ) targeting domain, a Bone Morphogenetic Protein (BMP) targeting domain, a Growth and Differentiation Factor (GDF) targeting domain, an activin targeting domain, or a Fibroblast Growth Factor (FGF) targeting domain, or a Platelet-Derived Growth Factor (PDGF) targeting domain.
10. The embodiments of 5 to 9, wherein the exogenous protease cleavage site is a plant papain cleavage site, an insect papain cleavage site, a crustacian papain cleavage site, an enterokinase cleavage site, a human rhinovirus 3C protease cleavage site, a human enterovirus 3C protease cleavage site, a tobacco etch virus protease cleavage site, a Tobacco Vein Mottling Virus cleavage site, a subtilisin cleavage site, a hydroxylamine cleavage site, or a Caspase 3 cleavage site.
11. The embodiments of 1 to 10, wherein the migraine disorder is a migraine without aura, a migraine with aura, a menstrual migraine, a migraine equivalent, a complicated migraine, an abdominal migraine, or a mixed tension migraine.
12. The embodiments of 1-11, wherein the TEM is administered to a nerve from the trigeminal nerve complex, or a nerve from the cervical nerve complex, a nerve from the spheno-palatine nerve complex, or a nerve from the trigeminal nerve complex, an olfactory nerve, a nerve innervating a cranial blood vessel, a nerve innervating a cervical blood vessel, or the brain.

### EXAMPLES

The following non-limiting examples are provided for illustrative purposes only in order to facilitate a more complete understanding of representative embodiments now contemplated. These examples should not be construed to limit any of the embodiments described in the present specification, including those pertaining to the compounds, compositions, methods or uses of treating a migraine disorder.

### Example 1

A 22 year old woman (occupation actress) presents with a history of headaches that are consistent with migraine. She has headaches on at least half the days of the month. These are felt over the fronto-temporal regions of the head bilaterally and to a lesser extent over the occipito-parietal areas. The pain is throbbing in nature. During the headache the scalp feels tender in these locations. Her headaches are associated with significant depression. She has failed to respond to numerous medications including treatment with botulinum toxin injected into the procerus, corrugator, frontalis, temporalis and occipitalis muscles. After signing a consent form she is treated with a TEM composition using the following injection technique.

The TEM composition is prepared according to the provided protocol. Two 1 cc syringes are prepared with an appropriate amount of TEM composition in each. The skull suture lines are palpated and mapped out. The hair is parted and the scalp cleaned with alcohol. Using a 1.5 inch, 27 gauge needle, the needle is inserted substantially parallel to the skull surface, along the suture lines. The first injection point is at the suture apex on the left side of her head, where the coronal suture and squamous suture meet. The needle is inserted upwardly first along the coronal suture and then gradually withdrawn as the plunger is depressed, so that a therapeutically effective amount is delivered in a linear and continuous fashion along the coronal suture on the left side of her head. The needle is then re-directed along the squamous suture line, using the same penetration point and the TEM composition is similarly administered. This is repeated on the right side of her head using the same method, so that a therapeutically effective amount is administered. Care is taken not to penetrate the periosteum, as this is known to cause an acute headache. The patient tolerates the procedure well and returns to the clinic 6 weeks later. Her headaches are lessened in frequency and intensity and her scalp is less tender. In addition she notes that her depression is alleviated.

### Example 2

A 37 year old chief financial officer arrives at his doctor's office complaining of headaches occurring about every three days over the past two months. The patient states that he experiences pain in the forehead and in the back of the head. The pain is described as a tight feeling, as if his head were in a vise. The physician decides to administer a TEM composition in the vicinity of and along the patient's coronal suture and the lambdoid suture. The TEM composition is prepared according to the provided protocol. Two 1 cc syringes are prepared with an appropriate amount of TEM composition in each. The skull suture lines, here the patient's coronal suture and lambdoid suture, are palpated and mapped out. The patient's hair is parted and the scalp cleaned with alcohol. Using a 1.5 inch, 27 gauge needle, the needle is inserted substantially parallel to the skull surface and laterally down along first the left and then right side of the skull, along the coronal suture. As previously described, the needle, in each instance (left and right side) is gradually withdrawn as the plunger is depressed, so that a therapeutically effective amount of the TEM composition is delivered in a linear and continuous fashion along and in the vicinity of the coronal suture.

Similarly, the patient's lambdoid suture is mapped out, the hair parted and the needle of a syringe containing a therapeutically effective amount of the TEM composition is inserted substantially parallel to the skull surface and downwardly along the left side of the patient's skull, along the lambdoid suture to its full needle length. The needle is gradually withdrawn as the plunger is depressed, so that a therapeutically effective amount of the TEM composition is delivered in a continuous, linear fashion along and in the vicinity of lambdoid suture on the left side, and the needle is then re-directed along the lambdoid suture line, this time to the right side of the skull, using the same procedure as with the left side.

The patient returns to the doctor's office two months later for a follow-up session. The patient states that since receiving the TEM composition administration along his suture lines, he has experienced only two headaches in the two months and these two headaches were of shorter duration and intensity when compared to his previously experienced headaches. The patient also reports that the nausea associated with previous headaches has been greatly reduced.

Groupings of alternative embodiments, elements, or steps of the present invention are not to be construed as limitations. Each group member may be referred to and claimed individually or in any combination with other group members disclosed herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Unless otherwise indicated, all numbers expressing a characteristic, item, quantity, parameter, property, term, and so forth used in the present specification and claims are to be understood as being modified in all instances by the term "about." As used herein, the term "about" means that the characteristic, item, quantity, parameter, property, or term so qualified encompasses a range of plus or minus ten percent above and below the value of the stated characteristic, item, quantity, parameter, property, or term. Accordingly, unless indicated to the contrary, the numerical parameters set forth in the specification and attached claims are approximations that may vary. At the very least, and not as an attempt to limit the application of the doctrine of equivalents to the scope of the claims, each numerical indication should at least be construed in light of the number of reported significant digits and by applying ordinary rounding techniques. Notwithstanding that the numerical ranges and values setting forth the broad scope of the invention are approximations, the numerical ranges and values set forth in the specific examples are reported as precisely as possible. Any numerical range or value, however, inherently contains certain errors necessarily resulting from the standard deviation found in their respective testing measurements. Recitation of numerical ranges of values herein is merely intended to serve as a shorthand method of referring individually to each separate numerical value falling within the range. Unless otherwise indicated herein, each individual value of a numerical range is incorporated into the present specification as if it were individually recited herein.

The terms "a," "an," "the" and similar referents used in the context of describing the present invention (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the present invention and does not pose a limitation on the scope of the invention otherwise claimed. No language in the present specification should be construed as indicating any non-claimed element essential to the practice of the invention.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the present invention so claimed are inherently or expressly described and enabled herein.

All patents, patent publications, and other publications referenced and identified in the present specification are individually and expressly incorporated herein by reference in their entirety for the purpose of describing and disclosing, for example, the compositions and methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

## Claims

1. A method for reduction of pain associated with a headache in a human in need thereof, comprising the step of administering a therapeutically effective amount of a Targeted Exocytosis Modulator (TEM) along the trajectory of an extracranial artery and to a sphenopalatine ganglion, wherein the extracranial artery is selected from the group consisting of a temporal artery and an occipital artery, thereby effecting reduction of headache pain in the human in need thereof.

2. The method of claim 1, wherein the TEM is administered by injection.

3. The method of claim 1 or 2, wherein the headache is selected from the group consisting of migraine, trigeminal autonomic cephalgia and headache caused by a vascular condition.

4. The method of claims 1-3, further comprising the step of locating the extracranial artery.

5. The method of claim 4, wherein the step of locating the extracranial artery utilizes palpation.

6. The method of claims 1-5, wherein administration to the sphenopalatine is performed bilaterally.

7. The method of claim 1-6, wherein administration of botulinum toxin is to a plurality of locations adjacent the sphenopalatine ganglion.
